# EUROPÄISCHE PATENTSCHRIFT

(11) **EP 0 729 740 B1**
(45) Veröffentlichungstag und Bekanntmachung des Hinweises auf die Patenterteilung: **24.03.2004**
(21) Anmeldenummer: 96102504.6
(22) Anmeldetag: 20.02.1996
(51) Int. Cl.: A61K 6/04

(54) **Verwendung von Goldlegierungen für Konstruktionselemente in der Dentaltechnik**
Use of gold alloys for making construction elements in dentistry
Utilisation d'alliages d'or pour la fabrication d'éléments de construction en art dentaire

(30) Priorität: 25.02.1995 DE 19506680; 12.02.1996 DE 19604827
(43) Veröffentlichungstag der Anmeldung: 04.09.1996
(73) Patentinhaber: DeguDent GmbH, 63457 Hanau (DE)
(72) Erfinder: Kempf, Bernd, Dr., 63839 Kleinwallstadt (DE); Ringelstein, Hans-Martin, 60358 Frankfurt/M. (DE); Meier, Bernd, Dr., 63571 Gelnhausen (DE); Meiers, Willi, 63755 Alzenau (DE)
(74) Vertreter: Stoffregen, Hans-Herbert, Dr. Dipl.-Phys.

(56) Entgegenhaltungen:
- EP-A- 0 691 123
- DE-A- 4 306 542
- DE-C- 4 419 408

## Beschreibung

Die Erfindung betrifft die Verwendung von Goldlegierungen als Werkstoff für Konstruktionselemente in der Dentaltechnik.

Speziell bei der Herstellung von herausnehmbarem Zahnersatz kommen häufig Präzisionsverbindungselemente als sogenannte Konstruktionselemente, wie zum Beispiel Geschiebe oder Gelenke zum Einsatz. Solche Konstruktionselemente werden heutzutage meist in vorgefertigter Form angeboten. Aufgrund der individuellen Problemfälle existiert eine Vielzahl unterschiedlicher Konstruktionen. So waren bereits 1989 ca. 290 verschiedene Systeme auf dem Markt. Zu ihrer Herstellung wird eine Vielzahl unterschiedlicher Legierungen auf Edelmetallbasis eingesetzt.

In der Regel sind diese Konstruktionselemente sehr aufwendig zu fertigen. Dies liegt an der Kleinheit der Teile, die gepaart ist mit komplizierten Geometrien und engen Toleranzen, um den hohen Ansprüchen im klinischen Einsatz standzuhalten. Zusätzlich müssen aufgrund der hohen Festigkeitsanforderungen hochfeste Legierungen verwendet werden, die in aller Regel aufgrund der hohen Härte ein schwieriges Umformverhalten zeigen. Üblicherweise werden nur Legierungen eingesetzt, die Härten von über 200 HV und Fließgrenzen von mehr als 450 MPa besitzen.

Zusätzlich werden an diese Legierungen hohe Anforderungen bezüglich der Korrosionsbeständigkeit gestellt, um die Biokompatibilität entsprechender Dentalkonstruktionen sicherzustellen. Dabei sind die Anforderungen bezüglich Korrosionsfestigkeit eher noch höher einzustufen als bei gegossenem Zahnersatz, da bei Konstruktionselementen Spalträume, zum Beispiel zwischen den Geschiebeflächen, nicht zu umgehen sind. Damit sind alle Vorraussetzungen für eine verschärfte Korrosionssituation durch Spaltkorrosion erfüllt. Eine optimale Biokompatibilität erhält man durch den Einsatz höchst korrosionsbeständiger Legierungen, die mit möglichst wenigen Legierungselementen auskommen.

Die Befestigung der Konstruktionselemente an die Prothesenteile kann durch Löten oder durch direktes Angießen der Dentallegierung an das Konstruktionselement erfolgen. Für diese Konstruktionselemente benötigt man Legierungen, die keine Nichtedelmetalle enthalten, so daß beim Vorwärmen vor dem Anguß keine störende Oxidschicht entsteht, die eine stoffschlüssige Verbindung zwischen Konstruktionselement und Dentallegierung verhindern würde. Diese Legierungen sind meist auf der Basis Gold-Platin-Palladium oder auch Platin-Iridium aufgebaut. Besonders hochfeste Legierungen dieses Typs sind beispielsweise in der DE-PS 35 42 641 beschrieben.

Speziell bei gelben, aufbrennfähigen Dentallegierungssystemen, die mit speziellen niedrigschmelzenden Keramiken verblendet werden können, sind Konstruktionselemente aus gelben Legierungen erwünscht, damit sich diese vom Grundmaterial nicht farblich abheben. Diese gelben Legierungen sind bisher alle mit Nichtedelmetallen legiert und damit nicht angußfähig. Sie sind in der Regel auf der Basis Gold-Platin-Silber-Kupfer aufgebaut und verdanken ihre mechanische Festigkeit zum großen Teil der Silber-Kupfer-Mischungslücke. Durch den dadurch bedingten relativ hohen Kupfer-Anteil ist potentiell eine Verfärbungsneigung speziell bei Vorliegen einer Spaltkorrosionssituation gegeben. In jüngerer Zeit hat man daher das Ziel verfolgt, den Kupfer-Gehalt dieser Legierungen weiter zu reduzieren. Um hohe mechanische Festigkeiten sicherstellen zu können, sind weitere legierungstechnische Maßnahmen erforderlich, die allerdings als Konsequenz eine Verringerung der Duktilität und damit einhergehend einen noch höheren Fertigungsaufwand zur Folge haben.

Aus der nicht vorveröffentlichten, jedoch prioritätsälteren DE 44 19 408 C1 ist eine Dental-Gold-Legierung für Zahnersatz bekannt, die 95 bis 98 % Gold, 1 bis 4 % Titan sowie 0,05 bis 1,5 % eines oder einer Mischung der Elemente Re, Rh, Ru, Ir und Ta enthält.

Die ebenfalls prioritätsältere nicht vorveröffentlichte EP 0 691 123 A1 beschreibt eine hochgoldhaltige Dentallegierung für Zahnersatz wie Kronen, Brücken usw. und enthält 91 bis 99,4 % Gold und 0,5 bis 3 % Titan, wobei zusätzlich ein sehr kleiner Anteil von Nichtedelmetallen enthalten ist.

Die DE 43 06 542 A1 beschreibt die Verwendung von Gold/Titan-Legierungen als Dentallegierungen, wobei 50 bis 91 Massen-% Gold und 50 bis 91 Massen- % Titan enthalten sein können.

Es war daher Aufgabe der vorliegenden Erfindung, Goldlegierungen für Konstruktionselemente in der Dentaltechnik zu finden, die eine gelbe Goldfarbe zeigen, ausreichend hart und gut verformbar sind. Außerdem sollten sie äußerst korrosionsbeständig sein, eine optimale Biokompatibilität besitzen und daher keine toxisch bedenklichen Bestandteile aufweisen.

Diese Aufgabe wird erfindungsgemäß gelöst durch die Verwendung von Goldlegierungen mit 1,2 bis 2,3 Gew.% Titan, Rest Gold.

Vorzugsweise verwendet man Legierungen, die 1,6 bis 1,8 Gew.% Titan, Rest Gold enthalten.

Überraschenderweise lassen sich auf Goldbasis mit Titanzusätzen von 1,2 bis 2,3 Gew.% Legierungen erzeugen, die ein wesentlich günstigeres Umform- und Korrosionsverhalten aufweisen, als die bisher bekannten Legierungen für Konstruktionselemente. Die hohe Reaktivität des Elementes Titan läßt sich schmelztechnisch durch Aufschmelzen unter Schutzgas in geeigneten Tiegeln beherrschen. Besonders bewährt haben sich binäre Legierungen aus Gold und 1,6 bis 1,8 Gew.% Titan, die bezüglich Farbe und Korrosionsbeständigkeit sowie Verformungsverhalten optimale Eigenschaften aufweisen.

In Tabelle 1 sind beispielhaft drei gelbe Legierungen des konventionellen Legierungstyps (Legierung 1 bis 3) neben einigen erfindungsgemäßen Legierungen (Legierungen 4 bis 6) aufgelistet. In Tabelle 2 finden sich die dazugehörigen mechanischen Kenndaten.

**Tabelle 1:**

| Legierungszusammensetzungen (Gew.%) | | | | | | | | | |
|---|---|---|---|---|---|---|---|---|---|
| Leg. | Au | Ag | Pt | Pd | Cu | Zn | In | Ti | Sonst. |
| 1 | 65.5 | 13 | 8.9 | 1 | 11,5 | 0.5 | | | Ir0.1 |
| 2 | 73.8 | 9.2 | 9 | - | 4.4 | 2 | 1.5 | | Ir0.1 |
| 3 | 97.0 | | | | | | | | Zr3.0 |
| 4 | 98.6 | | | | | | | 1.4 | Ir0.1 |
| 5 | 98.3 | | | | | | | 1.7 | |
| 6 | 97.9 | | | | | | | 2.1 | |

**Tabelle 2:**

| Mechanische Kenndaten | | | | | | |
|---|---|---|---|---|---|---|
| Leg. | HV-H | HV-W | Rp-H (MPa) | Rp-W (MPa) | A-W (%) | A-H (%) |
| 1 | 275 | 180 | 710 | 420 | 28 | 11 |
| 2 | 230 | 150 | 500 | 310 | 14 | 6 |
| 3 | 230 | 90 | 600 | 247 | 21 | <1 |
| 4 | 210 | 145 | 420 | 82 | 71 | 12 |
| 5 | 240 | 55 | 480 | 95 | 61 | 9 |
| 6 | 250 | 95 | 500 | 170 | 45 | 6 |
| HV-H: Vickershärte im harten Zustand (jeweils optimale Vergütungstemperatur) | | | | | | |
| HV-W: Vickershärte im weichen Zustand (in Wasser abgeschreckt) | | | | | | |
| Rp-H: Fließgrenze im harten Zustand | | | | | | |
| Rp-W: Fließgrenze im weichen Zustand | | | | | | |
| A-H: Duktilität für harten Zustand im Zugversuch (techn. Dehnung) | | | | | | |
| A-W: Duktilität für weichen Zustand im Zugversuch (techn. Dehnung). | | | | | | |

Während die bekannten und die erfindungsgemäßen Legierungen. im harten Zustand keine gravierenden Unterschiede bei den mechanischen Kenndaten aufweisen, zeigen die erfindungsgemäßen Legierungen im weichgeglühten Zustand deutlich bessere Werte. Die Härtewerte lassen sich im Gegensatz zu den konventionellen Legierungen wesentlich stärker absenken, z.T. auf etwa nur ein Drittel der Werte, die die konventionellen Legierungen besitzen. Damit einher geht eine starke Absenkung der Fließgrenze und eine massive Steigerung der Duktilität auf Werte von ca. 60 %. Sowohl die Erhöhung der Duktilität als auch die deutliche Verringerung von Härte und Fließgrenze führen zu einer markanten Verbesserung des Umformverhaltens. Durch die höhere Duktilität lassen sich größere Umformgrade und eine Verringerung der Zahl der Zwischenglühungen erreichen und durch die geringere Festigkeit sind geringere Umformkräfte erforderlich. Beide Faktoren gemeinsam führen zu deutlich verringerten Fertigungskosten und -zeiten und erlauben außerdem neuartige, effizientere Fertigungsmethoden, wie zum Beispiel das Fließpressen. Nach den Umformvorgängen lassen sich durch geeignete Wärmebehandlungen wieder hohe Festigkeiten und geringere Duktilitäten einstellen. Der nunmehr harte Zustand besitzt ein sehr günstiges Verhalten bei nachfolgenden spanenden Formgebungen. Auch bei der Verwendung als Konstruktionselemente sind die hohen Festigkeiten wieder erforderlich.

Überraschenderweise hat sich außerdem gezeigt, daß diese Legierungen sich durch eine außergewöhnliche Korrosionsbeständigkeit auszeichnen. In Tabelle 3 sind die Kenndaten zur Korrosionsbeständigkeit zusammengestellt. Die Korrosionsbeständigkeit wurde nach DIN E 13927 in einem einwöchigen Immersionstest in 0,1 molarer Milchsäure-Kochsalzlösung anhand der freiwerdenden Korrosionsprodukte bestimmt. In Tabelle 3 sind die Summen der freiwerdenden Korrosionsprodukte pro cm² Probenoberfläche aufgelistet. Die Untersuchungen erfolgten einmal an Proben mit frisch geschliffener Oberfläche und einmal an Proben, die nachträglich noch einer Vergütungsbehandlung ausgesetzt waren. Als Vergütungsbehandlung wurde jeweils eine Auslagerung von 1h bei 500° C an Luft gewählt. Bei dieser Wärmebehandlung erreichen die erfindungsgemäßen Legierungen die höchsten Festigkeiten. Es bietet sich daher an, nach der letzten Formgebung eine solche Behandlung durchzuführen. Während es bei den konventionellen Legierungen dadurch zu einem Anstieg der Korrosionsrate kommt, so daß die Glühung unter Schutzgas durchgeführt werden muß, führt diese Glühbehandlung überraschenderweise bei den erfindungsgemäßen Legierungen zu einer weiteren Verbesserung der Korrosionsbeständigkeit. Bei den binären Legierungen sinkt die Korrosionsrate sogar unter die Nachweisgrenze ab.

| Legierung | Gesamt-Korrosionsrate (µg/cm²) geschliffene Oberfläche | Gesamt-Korrosionsrate (µg/cm²) nach zusätzlicher Vergütung |
|---|---|---|
| 1 | 6 | 43 |
| 2 | 8 | 32 |
| 5 | 0,3 | <N.G. (N.G. = Nachweisgrenze = 0,13 µg/cm²) |
| 6 | 0,5 | <N.G. |

Folgende Beispiele sollen die Verwendung der erfindungsgemäßen Legierungen näher erläutern:
1. Zur Herstellung eines Geschiebes muß ein Vormaterial mit einem Rechteckquerschnitt von 3,3 x 6 mm aus einem stranggegossenren Zylinderstab mit 9 mm Durchmesser hergestellt werden. Für Legierung 1 (Tabelle 1) sind dafür 8 Zwischenglühungen sowie 9 Walzstiche gefolgt von 3 Ziehschritten erforderlich.
   Für Legierung 2 (Tabelle 1) sind dafür 11 Zwischenglühungen sowie 10 Walzstiche gefolgt von 3 Ziehschritten erforderlich.
   Die erfindungsgemäßen Legierungen Nr. 5 und 6 (Tabelle 1) benötigen dagegen nur 3 bzw. 4 Zwischenglühungen sowie 6 Walzstiche gefolgt von 3 Ziehschritten. Die Fertigung des Vormaterials ist damit wesentlich weniger zeitaufwendig.
Zur Herstellung eines zylindrischen Wurzelstiftes werden konventionelle Legierungen auf einer Drehbank bearbeitet. Mit der erfindungsgemäßen Legierung Nr. 6 kann dagegen ein Draht angemessener Dicke nach vorhergehender Weichglühung direkt durch einen Fließpreßvorgang in eine Endform gepreßt werden. Eine Nacharbeitung ist für diesen Anwendungsfall nicht erforderlich.

## Patentansprüche

1. Verwendung von Goldlegierungen mit 1,2 bis 2,3 Gew.% Titan, Rest Gold, als Werkstoff für Konstruktionselemente in der Dentaltechnik.

2. Verwendung einer Legierung gemäß Anspruch 1, **dadurch gekennzeichnet,**
**daß** sie 1,6 bis 1,8 Gew.% Titan, Rest Gold enthält.

## Claims

1. Use of gold alloys containing 1.2 to 2.3 wt.% of titanium, the remainder being gold, as a material for construction elements in dental technology.

2. Use of an alloy according to Claim 1, **characterized in that** it contains 1.6 to 1.8 wt.% of titanium, the remainder being gold.

## Revendications

1. Utilisation d'alliages d'or comprenant 1,2 à 2,3 % en poids de titane, le reste étant de l'or, comme matériau destiné à des éléments de construction dans la technique dentaire.

2. Utilisation d'un alliage selon la revendication 1,
**caractérisé en ce qu'**
il comprend 1,6 à 1,8 % en poids de titane, le reste étant de l'or.
